# EUROPÄISCHE PATENTANMELDUNG

(11) **EP 3 199 575 A1**
(43) Veröffentlichungstag der Anmeldung: **02.08.2017**
(21) Anmeldenummer: 16153363.3
(22) Anmeldetag: 29.01.2016
(51) Int. Cl.: C08J 3/24, C07D 335/00, C07C 327/36, C07C 57/00, C07D 335/02

(54) **NEUARTIGER HETERO-DIELS-ALDER-VERNETZER UND DEREN VERWENDUNG IN REVERSIBEL VERNETZENDEN POLYMERSYSTEMEN**

(71) Anmelder: Evonik Degussa GmbH, 45128 Essen (DE)
(72) Erfinder: SCHMIDT, Friedrich Georg, 45721 Haltern am See (DE); PAULMANN, Uwe, 59348 Lüdinghausen (DE); RICHTER, Christian, 48151 Münster (DE); INHESTERN, Marcel, 45665 Recklinghausen (DE); MEIER, Christian, 64293 Darmstadt (DE); BARNER-KOWOLLIK, Christopher, 76297 Stutensee (DE); PAHNKE, Kai, 76137 Karlsruhe (DE); SANZ, Miguel Angel, 44225 Dortmund (DE); UMBREEN, Sumaira, 44225 Dortmund (DE); JANSSEN, Christian Ewald, 45657 Recklinghausen (DE)

(57) **Zusammenfassung**

Die Erfindung betrifft einen neuartigen Hetero-Diels-Alder-Vernetzer, dessen Verfahren zur Herstellung und seine Verwendung für reversibel vernetzende Polymersysteme.

## Beschreibung

### Gebiet der Erfindung

Die Erfindung betrifft einen neuartigen Hetero-Diels-Alder-Vernetzer, dessen Verfahren zur Herstellung und seine Verwendung für reversibel vernetzende Polymersysteme.

Anwendungen für diesen Vernetzer-Baustein und seine Derivate finden sich zur Herstellung von reversibel vernetzenden Polymersystemen für Spitzgieß- und Extrusionsformmassen, zur Herstellung von Schäumen, für Anwendungen im Bereich des Additive Manufacturing, wie z.B. mit dem SLS- oder dem FDM-Verfahren, für die Herstellung von Composite-Bauteilen nach dem RTM-Verfahren, zur Herstellung von lagerstabilen Prepregs und daraus hergestellten Formkörpern oder Composite-Bauteilen und auch für Klebstoffe und Beschichtungen. Die vorliegende Erfindung betrifft eine neuartige Methode zur reversiblen Vernetzung von beispielsweise Kleb- oder Beschichtungsmassen.

Die reversible Vernetzungsmethode ermöglicht dabei eine sehr schnelle Vernetzung schon bei Raumtemperatur und eine Lösung der Vernetzungsstellen bei höheren Temperaturen, so dass eine thermoplastische Verarbeitbarkeit zurück gewonnen wird und z.B. die ursprünglich verklebten Substrate leicht wieder voneinander getrennt werden können. Dabei ist ein besonderer Aspekt, dass mehrere Zyklen einer Vernetzung und einer Lösung der Vernetzungsstellen mit dem vorliegenden System möglich sind.

### Stand der Technik

Methoden zur reversiblen Vernetzung von Polymeren sind für ein breites Feld von Anwendungen von großem Interesse. Beispielsweise in Klebanwendungen sind diverse Möglichkeiten für die Automobilindustrie oder die Halbleiterindustrie beschrieben. Aber auch bei der Konstruktion von Maschinen, feinmechanischen Geräten oder in der Bauinduistrie sind solche Klebstoffe interessant. Neben Klebanwendungen können reversibel vernetzbare Polymere auch in Dichtstoffen, Beschichtungsmassen wie Lacken oder Farben oder bei der Herstellung von Formkörpern interessant sein.

In DE 198 32 629 und in DE 199 61 940 sind Verfahren beschrieben, bei dem Klebstoffe auf Epoxy-, Harnstoff-, (Meth)acrylat- oder Isocyanatbasis thermisch zersetzt werden. Die Klebstoffformulierung aus DE 199 61 940 enthält dazu eine thermisch instabile Substanz, die bei Erhitzen aktiviert wird. Die Klebschicht in DE 198 32 629 wird durch besonders hohen Energieeintrag zerstört. Die Desaktivierung der Klebschicht ist in beiden Fällen irreversibel.
In US 2005/0159521 bzw. in US 2009/0090461 ist ein Klebsystem beschrieben, das durch Bestrahlung mit aktinischer Strahlung radikalisch vernetzt und durch Ultraschall zerstört wird. Auch dieses Verfahren ist nach einem Klebezyklus irreversibel nicht mehr durchführbar.
In EP 2 062 926 sind in die Ketten eines Polyurethans für Klebanwendungen thermisch labile, sterisch gehinderte Harnstoffgruppen eingebaut, die durch Eintrag thermischer Energie zerstört wird und damit die Klebwirkung ausreichend zur Lösung der Verbindung reduziert wird.
In US 2009/0280330 ist ein wahrscheinlich mehrfach verwendbares Klebsystem beschrieben, das einen Zweischichtaufbau aufweist. Bei einer Schicht handelt es sich um eine Shape-Memory-Schicht, die thermisch flexibel oder gehärtet werden kann. Bei der anderen Schicht handelt es sich um einen Trockenkleber, der unterschiedliche Klebstärken in Abhängigkeit von der Struktur aufweist. Problem eines solchen Systems ist jedoch die aufwendig aufzubauende zweischichtige Struktur und die zu erwartende Restklebrigkeit nach Erhitzen der Shape-Memory-Schicht.

Unter dem Oberbegriff "Click Chemie" werden seit einigen Jahren vor allem in der akademischen Welt Methoden zum Aufbau von Blockcopolymeren erforscht. Dabei werden zwei unterschiedliche Homopolymere mit verknüpfbaren Endgruppen miteinander kombiniert und z.B. mittels einer Diels-Alder-Reaktion, Diels-Alder-analogen Reaktion bzw. einer anderen Cycloaddition miteinander verbunden. Ziel dieser Reaktion ist es, thermisch stabile, lineare und ggf. hochmolekulare Polymerketten aufzubauen. In Inglis et al. (Macromolecules 2010, 43, S.33-36) beispielsweise sind zu diesem Zweck Polymere mit Cyclopentadienylendgruppen, die aus mittels ATRP hergestellten Polymeren erhältlich sind, beschrieben. Diese Cyclopentadiengruppen können sehr rasch in hetero Diels-Alder Reaktionen mit Polymeren reagieren, welche elektronenarme Dithioester als Endgruppen tragen (Inglis et al. Angew. Chem. Int. Ed. 2009, 48, S. 2411-2414).
Die Verwendung von monofunktionellen RAFT-Polymeren zur Verknüpfung mit monofunktionellen Polymeren, die eine Dihydrothiopyran-Gruppe über eine Hetero-Diels-Alder-Reaktion findet sich in Sinnwell et al. (Chem. Comm. 2008, 2052-2054). Mit dieser Methode lassen sich AB-Diblockcopolymere realisieren. Schnelle Varianten dieser Hetero-Diels-Alder-Verknüpfung zur Synthese von AB-Blockcopolymeren mit einer nach einer RAFT-Polymerisation vorliegenden Dithioester-Gruppe und einer Dienyl-Endgruppe sind in Inglis et al. (Angew.Chem.Int.Ed. 2009, 48, S.2411-14) und in Inglis et al. (Macromol. Rapd Commun. 2009, 30, S.1792-98) beschrieben. Die analoge Herstellung von Multiarm-Sternpolymeren findet sich in Sinnwell et al. (J.Pol.Sci.: Part A: Pol.Chem. 2009, 47, S.2207-13).

In US 6,933,361 ist ein System zur Herstellung von einfach reparierbaren, transparenten Formkörpern beschrieben. Das System besteht aus zwei multifunktionellen Monomeren, die mittels einer Diels-Alder-Reaktion zu einem hochdichten Netzwerk polymerisieren. Dabei handelt es sich bei der einen Funktionalität um ein Maleinsäureimid und bei der anderen Funktionalität um ein Furan. Das thermische Schalten eines solchen hochdichten Netzwerks dient zur Reparatur desselbigen. Die Vernetzung findet bei Temperaturen oberhalb von 100 °C statt. Die partielle Rückreaktion bei noch höheren Temperaturen.

Bei Syrett et al. (Polym.Chem. 2010, DOI: 10.1039/b9py00316a) werden Sternpolymere zur Verwendung als Fließverbesserer in Ölen beschrieben. Diese weisen mittels einer reversiblen Diels-Alder-Reaktion steuerbare, selbstheilende Eigenschaften auf. Dazu werden monofunktionelle Polymethacrylatarme mit Polymethacrylaten kombiniert, die in der Mitte der Kette, als Fragment des eingesetzten Initiators über eine in einer reversiblen Diels-Alder-Reaktion verwendbare Gruppe verfügen.

In der EP 2 536 797 ist ein reversibel vernetzendes System aus zwei Komponenten A und B bestehend offenbart. Dabei handelt es sich bei Komponente A um eine Verbindung mit mindestens zwei dienophilen Gruppen und bei Komponente B um eine Verbindung mit mindestens zwei Dienfunktionalitäten. Die in der EP 2 536 797 offenbarten Kombinationen aus den Komponenten A und B sind in Bezug auf die Maximalzahl möglicher Schaltzyklen und die Lagerstabilität der Zusammensetzungen durchaus noch optimierbar.

Insbesondere für Anwendungen reversibel vernetzender Systeme im technischen Gebiet der Compositmaterialien ist darüber hinaus weiterer Stand der Technik von Bedeutung. Faserverstärkte Materialien in Form von Prepregs werden bereits in vielen industriellen Anwendungen wegen ihrer bequemen Handhabung und der erhöhten Effizienz bei der Verarbeitung im Vergleich zu der alternativen wet-layup Technologie eingesetzt.
Industrielle Anwender solcher Systeme verlangen neben schnelleren Zykluszeiten und höheren Lagerstabilitäten - auch bei Raumtemperatur - zusätzlich eine Möglichkeit, die Prepregs zuzuschneiden, ohne dass bei automatisiertem Zuschnitt und Lay-up der einzelnen Prepreg-Lagen, die Schneidwerkzeuge mit der häufig klebrigen Matrixmaterial verunreinigt werden. Verschiedene Formgebungsprozesse, wie z.B. das Reaction-Transfer-Moulding-(RTM)-Verfahren beinhalten die Einbringung der Verstärkungsfasern in eine Form, das Schließen der Form, das Einbringen der vernetzbaren Harzformulierung in die Form und die anschließende Vernetzung des Harzes, typischerweise durch Wärmezufuhr.
Eine der Beschränkungen eines solchen Prozesses ist das relativ schwierige Einlegen der Verstärkungsfasern in die Form. Die einzelnen Lagen des Gewebes oder Geleges müssen zugeschnitten und den unterschiedlichen Geometrien der jeweiligen Formteile angepasst werden. Das kann sowohl zeitintensiv wie auch kompliziert sein, insbesondere wenn die Formkörper auch Schaum-oder andere Kerne enthalten sollen. Vorformbare Faserverstärkungen mit einfachem Handling und bestehenden Umformmöglichkeiten wären hier wünschenswert.
Neben Polyestern, Vinylestern und Epoxy-Systemen gibt es eine Reihe spezieller Harze im Bereich der vernetzenden Matrix-Systeme. Dazu zählen auch Polyurethan-Harze, die wegen ihrer Zähigkeit, Schadenstoleranz und die Festigkeit insbesondere zur Herstellung von Composite-Profilen über Pultrusionsverfahren eingesetzt werden. Als Nachteil wird häufig die Toxizität der verwendeten Isocyanate genannt. Jedoch auch die Toxizität von Epoxy-Systemen und der dort verwendeten Härter-Komponenten ist als kritisch anzusehen. Dies gilt insbesondere für bekannte Sensibilisierungen und Allergien.
Darüber hinaus haben die meisten Matrixmaterialien zur Herstellung von Prepregs für Composites den Nachteil, dass sie bei der Applikation auf das Fasermaterial entweder in fester Form, z.B. als Pulver, oder als hochviskose Flüssigkeit oder Schmelze vorliegen. In beiden Fällen erfolgt eine nur geringe Durchtränkung des Fasermaterials mit dem Matrixmaterial, was wiederum zu einer nicht optimalen Stabilität des Prepregs bzw. des Composite-Bauteils führen kann.
Prepregs und daraus hergestellte Composites auf der Basis von Epoxy-Systemen werden zum Beispiel in WO 98/50211, EP 0 309 221, EP 0 297 674, WO 89/04335 und US 4,377,657 beschrieben. In WO 2006/043019 wird ein Verfahren zur Herstellung von Prepregs auf der Basis von Epoxidharz-Polyurethanpulvern beschrieben. Des Weiteren sind Prepregs auf der Basis von pulverförmigen Thermoplasten als Matrix bekannt.
In WO 99/64216 werden Prepregs und Composite und eine Methode zu deren Herstellung beschrieben, bei der Emulsionen mit so kleinen Polymerpartikeln verwendet werden, dass eine Einzelfaserumhüllung ermöglicht wird. Die Polymere der Partikel haben eine Viskosität von mindestens 5000 centipoise und sind entweder Thermoplaste oder vernetzende Polyurethan-Polymere.
In der EP 0 590 702 werden Pulverimprägnierungen zur Herstellung von Prepregs beschrieben, bei denen das Pulver aus einem Gemisch aus einem Thermoplasten und einem reaktiven Monomer bzw. Prepolymeren besteht. Die WO 2005/091715 beschreibt ebenfalls die Verwendung von Thermoplasten zur Herstellung von Prepregs.
Prepregs, die unter Verwendung von Diels-Alder-Reaktionen und potentiell aktivierbaren Retro-Diels-Alder Reaktionen hergestellt wurden, sind gleichfalls bekannt. Bei A.M. Peterson et al. (ACS Applied Materials & Interfaces (2009), 1(5), 992-5) werden entsprechende Gruppen in Epoxy-Systemen beschrieben. Durch diese Modifizierung erhält man selbstheilende Eigenschaften der Bauteile. Analoge Systeme, die nicht auf einer Epoxy-Matrix beruhen, finden sich u.a. auch bei J.S. Park et al. (Composite Science and Technology (2010), 70(15), 2154-9) oder bei A.M. Peterson et al. (ACS Applied Materials & Interfaces (2010), 2(4), 1141-9). Keines der aufgeführten Systeme ermöglicht jedoch eine über eine Selbstheilung hinausgehende nachträgliche Modifizierung der Composites. Die klassische Diels-Alder-Reaktion ist unter den möglichen Bedingungen nur unzureichend zurückzuführen, so dass hier nur geringe Effekte, wie sie für eine Selbstheilung beschädigter Bauteile ausreichend sein können, möglich sind.
In der EP 2 174 975 und in EP 2 346 935 sind jeweils Composite Materialien, verwendbar als Laminat, mit bis-Maleinimid- und Furan-Gruppen beschrieben, welche thermisch recycelt werden können. Dem Fachmann ist leicht ersichtlich, dass ein solches System nur bei relativ hohen Temperaturen wieder aktiviert, d.h. zu mindestens einem großen Teil wieder entnetzt, werden kann. Bei solchen Temperaturen kommt es jedoch schnell zu weiteren Nebenreaktionen, so dass der Mechanismus - wie beschrieben - nur zum Recyceln, nicht jedoch zum Modifizieren der Composites geeignet ist.
In WO 2013/079286 werden Composite-Materialien bzw. Prepregs zu deren Herstellung beschrieben, die Gruppen für eine reversible Hetero-Diels-Alder-Reaktion aufweisen. Diese Systeme sind reversibel vernetzbar und die Formteile damit sogar recycelbar. Jedoch lassen sich diese Systeme nur als flüssiges 100%-System oder aus einer organischen Lösung applizieren. Damit ist die Anwendbarkeit dieser Technologie deutlich eingeschränkt.
Alle beschriebenen Systeme basieren entweder auf organischen Lösungsmitteln oder werden als Schmelze oder als flüssiges 100%-System appliziert. Keines der beschriebenen Systeme kann jedoch in Form einer wässrigen Dispersion appliziert werden. Insbesondere solche wässrigen Systeme hätten jedoch große Vorteile in Hinblick auf die Arbeitssicherheit und zusätzlich verfügbare Verarbeitungstechnologien bei der Herstellung von Prepregs bzw. Composite-Materialien.
In der EP 2 931 817 wurde die Herstellung und Verwendung eines neuartigen Vernetzers für die reversible Hetero-Diels-Alder-Vernetzung beschrieben. Der dort beschriebene Vernetzer zeichnet sich zwar durch eine schnelle Reaktion mit entsprechenden Dienen aus, muss allerdings bei der Synthese durch Cyclopentadien oder ähnlich Diene als Schutzgruppe stabilisiert werden. Der reine Vernetzer ist in Substanz auch nicht stabil.

### Aufgabe

Aufgabe der vorliegenden Erfindung ist die Bereitstellung einer neuen reversiblen Vernetzungsmethode, die in unterschiedlichen Anwendungen und in einem breiten Formulierungsspektrum einsetzbar ist.

Weiterhin bestand die Aufgabe, Vernetzer-Strukturen zu finden, die ohne Schutzgruppen thermisch so stabil sind, dass die Verwendung von Cyclopentadien oder ähnlichen Strukturen als Blockierungsmittel nicht erforderlich sind.

Darüber hinaus sollten die Syntheseschritte wie auch die erzielten Ausbeuten für eine wirtschaftliche Herstellung der vernetzenden Systeme zu einer einfachen und robusten Herstellungsmethode verbessert werden, wobei auch die Edukt-Kosten gegenüber aus dem Stand der Technik bekannten System gesenkt werden sollten.

Zusätzlich sollten die Retro-Diels-Alder-Reaktion bei Temperaturen stattfinden, die Glastemperaturen des Gesamtsystems von größer 100°C ermöglichen und gleichzeitig die Verarbeitungstemperaturen eines z.B. Methacrylat-basierten Systems nicht über 240 °C gesteigert werden müssen.

Weitere nicht explizit genannte Aufgaben ergeben sich aus dem Gesamtzusammenhang der nachfolgenden Beschreibung, Ansprüche und Beispiele.

### Lösung

Gelöst wurden die Aufgaben durch Entwicklung einer neuartigen Formulierung, geeignet zur Durchführung eines reversiblen Vernetzungsmechanismus, der unabhängig von den Formulierungsbestandteilen wie Bindemittel für verschiedenartige Polymere einsetzbar ist. Überraschend wurde gefunden, dass die gestellten Aufgaben durch eine neuartige Formulierung, die mittels einer Hetero-Diels-Alder-Reaktion vernetzbar ist, gelöst werden können.

Dabei enthält die neuartige, reversibel vernetzbare Formulierung, die mittels einer Hetero-Diels-Alder-Reaktion vernetzbar ist, eine Komponente A, die mindestens zwei dienophile Doppelbindungen aufweist, wobei die Komponente A mindestens einmal die folgende Struktureinheit Z 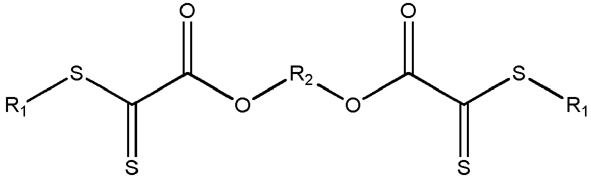 aufweist, wobei es sich bei R₁ um einen Alkyl- oder Alkylenrest mit 1 bis 20 Kohlenstoffatomen handelt, wobei der Alkylenrest mit weiteren der aufgezeigten Strukturen verbunden sein kann. Weiterhin enthält die Formulierung eine Komponente B, die mindestens eine Dien-Funktionalität aufweist.

Die reversible Vernetzung, die mit der erfindungsgemäßen Formulierung möglich ist, ermöglicht eine sehr schnelle Umsetzung schon bei einer niedrigen ersten Temperatur und eine Lösung der Vernetzungsstellen bei höheren Temperaturen, so dass eine thermoplastische Verarbeitbarkeit zurück gewonnen wird und z.B. die ursprünglich vernetzten Schichten bei einem Einsatz im Bereich der als Laminate verpressten Einzelschichten in Composites leicht wieder voneinander getrennt oder z.B. die vernetzten Einzelschichten, welche beispielsweise als Prepregs vorliegen, umgeformt und zu einem Laminat verpresst werden können.
Dabei ist ein besonderer Aspekt, dass mehrere Zyklen einer Vernetzung und einer Lösung der Vernetzungsstellen mit dem vorliegenden System möglich sind.

Die beschriebenen Vernetzer- bzw. Kettenverlängerungs-Moleküle sind als Reinstoffe bei TemperaturErhöhung so stabil, dass sie nicht mit Schutzgruppen blockiert werden müssen.

Die erfindungsgemäßen Formulierungen weisen insbesondere die im Folgenden genannten besonderen Vorteile auf:
- Es sind keine Schutzgruppen / Blockierungsgruppen für das reaktive Dienophil bei der Synthese erforderlich.
- Sehr einfache und robuste Synthese mit preiswerten Edukten und hoher Ausbeute
- Die Formulierung ist auch ohne Schutzgruppen temperaturstabil bis über 200°C
- Die Retro-Hetero-Diels-Alder-Reaktion erfolgt bei Temperaturen, die Schmelzpunkt bzw. Glastemperaturen des Gesamtsystems von größer 100°C ermöglichen.

Bevorzugt weist zumindest eine dieser beiden Komponenten A oder B mehr als zwei Funktionalitäten aufweist.
Gleichsam bevorzugt liegt mindestens eine der Komponenten A oder B als Polymer vor.
Auch bevorzugt ist die Formulierung bei Raumtemperatur vernetzbar. Dabei kann die Vernetzung bei einer höheren Temperatur zu mindestens 50% wieder rückgängig gemacht werden.

Die Komponente A ist beispielsweise über folgenden allgemeinen Syntheseweg verfügbar: 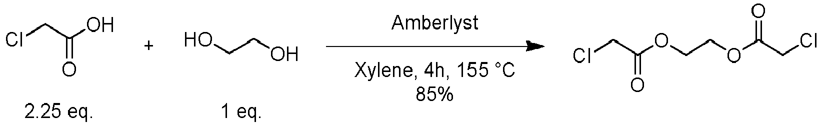 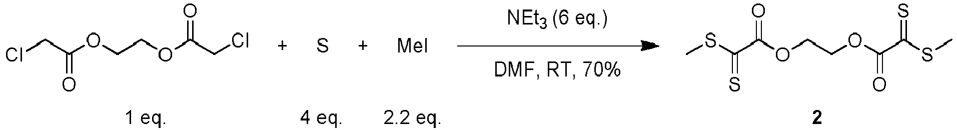 Anstelle des Ethylenglykols können für eine längere Alkylenkette (R₂) auch andere Diole, wie beispielsweise Hexandiol, eingesetzt werden.

Besonders bevorzugt handelt es sich bei Komponente A um eine Verbindung mit mehreren der genannten Struktureinheiten Z. Dabei handelt es sich insbesondere bevorzugt bei den Resten R₁ um Alkylengruppen, die zwischen 1 und 5 Kohlenstoffatomen aufweisen, über die die Struktureinheiten Z miteinander verbunden sind. Bevorzugt ist der R₂ dabei eine Alkylgruppe mit 2 bis 10 Kohlenstoffatomen.

Ganz besonders bevorzugt handelt es sich bei der Komponente A um die Verbindung 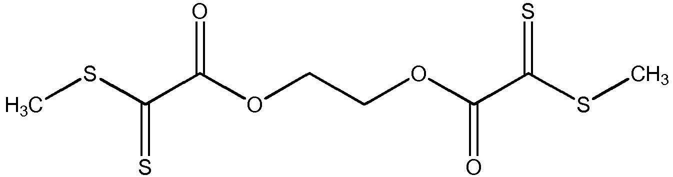 und/oder um die Verbindung 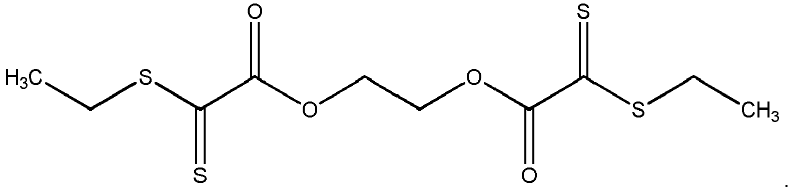 In einer besonderen Ausführungsform handelt es sich bei der Komponente B um ein Polymer. Bevorzugte Polymere sind dabei Polyacrylate, Polymethacrylate, Polystyrole, Mischpolymerisate aus Acrylaten, Methacrylaten und/oder Styrolen, Polyacrylnitril, Polyether, Polyester, Polymilchsäuren, Polyamide, Polyesteramide, Polyurethane, Polycarbonate, amorphe oder teilkristalline Poly-α-Olefine, EPDM, EPM, hydrierte oder nicht hydrierte Polybutadiene, ABS, SBR, Polysiloxane und/oder Block-, Kamm- und/oder Sterncopolymeren dieser Polymere.

Bezüglich Komponente B können von dem Fachmann geeignete Verbindungen, Dien-Funktionen, die für eine Hetero-Diels-Alder-Reaktion geeignet sind, aufweisend, relativ frei gewählt werden. Als besonders geeignet haben sich die folgenden drei Alternativen erwiesen:

In der ersten Alternative handelt es sich bei der Komponente B um eine der folgenden Verbindungen:

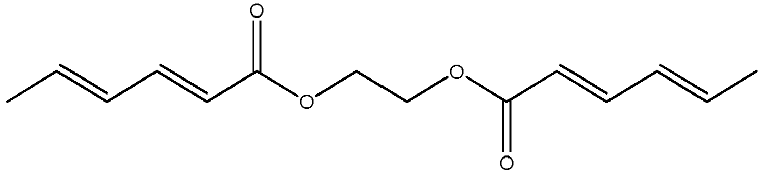

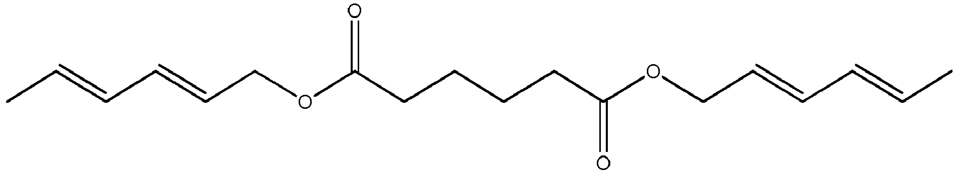

In einer zweiten Alternative handelt es sich bei der Komponente B um ein Polymer, welches unter Copolymerisation mindestens einer der folgenden Comonomere erhalten wurde:

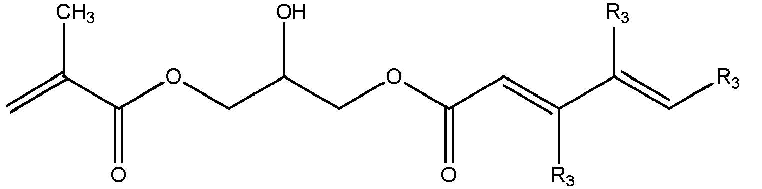

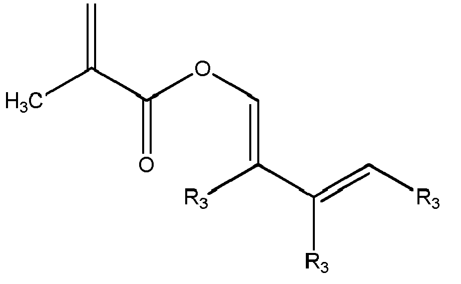

Dabei kann es sich bei den Resten R₃ um gleiche oder verschiedene Reste handeln. Bevorzugt ist R₃ Wasserstoff und/oder ein Alkylrest mit 1 bis 10 Kohlenstoffatomen.
Diese Monomere können beispielsweise mit (Meth)acrylaten und/oder Styrol copolymerisiert werden.

In der dritten bevorzugten Ausführungsform handelt es sich bei der Komponente B um ein Polyamid, einen Polyester oder ein Polycarbonat, aufweisend mindestens eine Dienfuntkionalität.

In einer besonderen Ausführungsform der vorliegenden Erfindung wird die erfindungsgemäße Formulierung nicht vernetzt, sondern es erfolgt eine Kettenverlängerung und damit eine Schaltung zwischen zwei unterschiedlichen thermoplastischen Zuständen. In einer solchen Formulierung weist die Komponente A genau eine Struktureinheit Z und Komponente B genau eine Diengruppe auf.

Die in diesem Text verwendete Schreibweise (Meth)acrylate steht für Alkylester der Acrylsäure und/oder der Methacrylsäure.

In einer weiteren möglichen Ausführungsform handelt es sich bei Komponente B um ein mittels Atomtransfer radikalsiche Polymersation (ATRP) hergestelltes, bifunktionelles Polymer. In diesem Fall erfolgt die Funktionalisierung mit den Diengruppen durch eine polymeranaloge oder während des Abbruchs durchgeführte Substitution endständiger Halogenatome. Diese Substitution kann beispielsweise durch Zugabe von mit Diengruppen funktionalisierten Mercaptanen erfolgen.

Ein weiterer Aspekt der vorliegenden Erfindung ist das Verfahren zur reversiblen Vernetzung der erfindungsgemäßen Formulierungen. Bei der Durchführung dieses Verfahrens wird eine Formulierung aus zumindest zwei unterschiedlichen Komponenten A und B, mittels einer Hetero-Diels-Alder-Reaktion bei Raumtemperatur vernetzt. In einem zweiten Verfahrensschritt werden bei einer höheren Temperatur mindestens 50%, bevorzugt mindestens 90% und besonders bevorzugt mindestens 99% der Vernetzungsstellen mittels einer Retro-Hetero-Diels-Alder-Reaktion wieder gelöst.

Bei Durchführung dieses zweiten Verfahrensschritts werden bei einer Temperatur oberhalb von 80 °C bevorzugt innerhalb von 5 min, höchstens innerhalb von 10 min, mindestens 90 Gew%, bevorzugt mindestens 95 Gew% und besonders bevorzugt mindestens 98 Gew% der Formulierung in einem für die Formulierung vor der Vernetzung geeigneten Lösungsmittel wieder löslich. Die Vernetzung zuvor war derart ausgeprägt, dass bei einem 5minütigem Auswaschen mit dem gleichen Lösungsmittel höchstens 5 Gew%, bevorzugt höchstens 2 Gew% und besonders bevorzugt höchstens 1 Gew% der Formulierung gelöst werden konnten. Der Ausdruck Formulierung und sämtliche damit verbundenen prozentualen Angaben beziehen sich in diesem Fall nur auf die Komponenten A und B. Weitere Formulierungsbestandteile, wie sie zum Beispiel in einer Beschichtungs- oder Klebstoffzusammensetzung zugegeben werden können, sind in dieser Betrachtung nicht berücksichtigt. Im Weiteren beschreibt der Ausdruck Formulierung im Rahmen dieser Schrift ausschließlich die Komponenten A und B sowie einen optionalen Vernetzungskatalysator. Der Ausdruck Zusammensetzung dagegen umfasst neben der Formulierung zusätzlich zugegebene Komponenten. Bei diesen zusätzlichen Komponenten kann es sich um spezifisch für die jeweilige Anwendung ausgesuchte Zuschlagsstoffe wie beispielsweise Füllstoffe, Pigmente, Additive, Verträglichkeitsvermittler, Cobindemittel, Weichmacher, Schlagzähmodifier, Verdicker, Entschäumer, Dispergieradditive, Rheologieverbesserer, Haftvermittler, Kratzfestadditive, Katalysatoren oder Stabilisatoren handeln.

Entsprechend der bereits beschriebenen Formulierung werden in dem Verfahren zuerst die Komponenten A und B, sowie optionale weitere Zuschlagsstoffe zusammen gebracht.

Die Vernetzungsreaktion kann bei Raumtemperatur innerhalb von 10 min, bevorzugt innerhalb von 5 min, besonders bevorzugt innerhalb von 2 min und ganz besonders bevorzugt innerhalb von einer Minute erfolgen. Zur Beschleunigung der Vernetzung kann nach dem Vermischen der Komponenten A und B ein Vernetzungskatalysator zugegeben werden. Bei diesen Vernetzungskatalysatoren handelt es sich in der Regel um starke Säuren wie Trifluoressigsäure oder Schwefelsäure oder um starke Lewis-Säuren wie z.B. Bortrifluorid, Zinkdichlorid, Titandichlorid-diisopropylat oder Aluminiumtrichlorid.

In einer alternativen Ausführungsform kann die Vernetzung auch ohne einen Katalysator, z.B. thermisch, beschleunigt werden. Dabei liegt die Aktivierungstemperatur unterhalb der Temperatur, die für die Retro-Hetero-Diels-Alder-Reaktion benötigt wird.

In einer weiteren alternativen Ausführungsform enthält die Formulierung unabhängig von der Aktivierung der Vernetzungsreaktion einen weiteren Katalysator, der die Aktivierungstemperatur der Retro-Hetero-Diels-Alder-Reaktion absenkt. Bei diesen Katalysatoren kann es sich beispielsweise um Eisen oder eine Eisenverbindung handeln.

Die erfindungsgemäßen Formulierungen bzw. Verfahren können in verschiedensten Anwendungsgebieten zur Verwendung kommen. Die nachfolgende Liste zeigt einige bevorzugte Anwendungsgebiete beispielhaft auf, ohne die Erfindung diesbezüglich in irgendeiner Form einzuschränken. Solche bevorzugten Anwendungsgebiete sind Klebstoffe, Dichtstoffe, Formmassen, Schäume, Lacke, Farbe, Beschichtungen, Öladditive - wie beispielsweise Fließverbesserer - oder Tinten.

Beispiele für Formmassen sind beispielsweise Polyester, Polycarbonat, Poly(meth)acrylate oder Polyamid, bei denen eine bei erhöhter Temperatur stattfindende Retro-Hetero-Diels-Alder-Entkupplung in Kombination mit der bei tieferer Temperatur sich wieder bildenden Polymerkettenverknüpfungen eine für den Kunststoff-Spritzguss vorteilhafte verringerte Viskosität bzw. erste entstehende Fließfähigkeit erst ermöglicht. Die bei tieferer Temperatur und sich beim Abkühlen wieder bildenden Polymerkettenverknüpfungen verbessern beispielsweise die mechanischen Eigenschaften des Formteils. Bei den Formmassen kann es sich im Allgemeinen beispielsweise um Spitzgieß- oder Extrusionsformmassen handeln.
Beispielsweise bei der Verwendung der gleichsam erfindungsgemäßen Formulierungen zur Kettenverlängerung können diese so in der Wärme oberhalb der Retro-Hetero-Diels-Alder-Temperatur - d.h. im "offenen" Zustand bei Spritzgieß-Prozessen - durch die geringe Viskosität der Schmelze a) feinere Strukturen besser abgebildet werden, b) mit niedrigeren Drücken und / oder c) dünnwandigere Teile hergestellt werden, die aber nach der Wiederverknüpfung die Eigenschaften von höhermolekulareren Polymeren zeigen.
Bei der Verwendung der erfindungsgemäßen Formulierungen unter Vernetzung können diese so in der Wärme oberhalb der Retro-Hetero-Diels-Alder-Temperatur d.h. im "offenen" Zustand überhaupt erst z.B. die für thermoplastische Werkstoffe üblichen Verarbeitungsverfahren wie z.B. Blasform-, Spritzgieß- oder Extrusions-Prozesse eingesetzt werden. Die nach der Wiedervernetzung erreichbaren Eigenschaften der vernetzten Polymeren zeigen eine deutliche erhöhte Performance, wie sie dem Fachmann auch bei z.B. durch Strahlenvernetzung irreversibel vernetzten Polymere bekannt sind.

Bei diesen Tinten handelt es sich beispielsweise um Zusammensetzungen, die thermisch appliziert werden und auf dem Substrat vernetzen. Wenn man leitfähige Oligomere verwendet oder Additive zur Erzeugung einer Leitfähigkeit im Allgemeinen, wird eine elektrisch leitende Tinte erhalten, die z.B. per Bubble-jet - Verfahren verarbeitet werden kann.

Beispiele aus den Anwendungsgebieten Lacke, Beschichtungen und Farbe sind Zusammensetzungen, die z.B. poröse Materialien im entnetzten Zustand besonders gut tränken oder benetzen können und in Folge der Vernetzungsreaktion hochkohärente Materialien ergeben.

Ähnliche Charakteristika sind von Bedeutung für Klebstoffe, die hohe Kohäsion aufweisen sollten und dennoch leicht die Oberflächen der zu klebenden Materialien benetzen sollen.
Eine weitere Anwendung im Bereich Kleben ist zum Beispiel eine nur temporär benötigte, später wieder zu lösende Fügung, wie sie in verschiedenen Produktionsprozessen zum Beispiel im Automobilbau oder im Maschinenbau vorkommen kann.
Eine andere denkbare Anwendung ist das Verkleben von Bauteilen, die über die Lebensdauer des gesamten Produktes gesehen eine hohe Austauschwahrscheinlichkeit haben, und daher möglichst einfach und rückstandsfrei wieder zu entfernen sein sollten. Ein Beispiel für eine solche Anwendung ist das Verkleben von Automobilfrontscheiben.
Ein besonderes Beispiel für Kleb- oder Dichtstoffe ist die Verwendung in Lebensmittelverpackungen, die sich beim Aufheizen, beispielsweise in einer Mikrowelle selbsttätig lösen lassen bzw. öffnen.

Ein Beispiel für Anwendungen im Bereich Rapid-Prototyping für die hier beschriebenen vernetzenden und entnetzenden Materialien sind im Bereich FDM (Fused Deposition Modeling), SLS (Selective Laser Sintering) oder beim 3D-Druck per Ink-jet-Verfahren mit niedrigviskosen Schmelzen zu finden.

Besonders bevorzugt ist die Anwendung der erfindungsgemäßen Formulierungen im Bereich Composites.
So können die erfindungsgemäßen Formulierungen beispielsweise als Dispersion für die Imprägnierung von Fasermaterial, wie z.B. Carbonfasern, Glasfasern oder Polymerfasern eingesetzt werden. Die so imprägnierten Fasern können dann wiederum zur Herstellung von Prepregs nach bekannten Verfahren verwendet werden.

Die Erfindung betrifft dabei beispielsweise auch Emulsionspolymerisate, die mit den erfindungsgemäßen Vernetzer-Molekülen der Formulierung mittels Hetero-Diels-Alder-Mechanismus intrapartikulär vernetzt werden. Die vernetzten Polymerisate können dann durch thermische Verarbeitung, beispielsweise als Composite-Matrix, durch eine Retro-Hetero-Diels-Alder-Reaktion ganz oder teilweise entnetzt und beim Abkühlen interpartikulär wieder vernetzt werden. Hierdurch sind über einen zweiten Weg lagerstabile Prepregs für Composite darstellbar. Aber auch andere Materialien, die duroplastische Eigenschaften bei Nutztemperatur haben, aber bei höherer Temperatur thermoplastische Verarbeitungseigenschaften besitzen, können so realisiert werden.

Bei Einsatz als Matrix-Werkstoff für Endlosfaserverstärkte Kunststoffe erhält man so Composite-Halbzeuge mit gegenüber dem Stand der Technik verbesserten Verarbeitungseigenschaften, die für die Herstellung leistungsfähiger Composite für verschiedenste Anwendungen im Bereich der Bau-, der Automobil-, der Luft- und Raumfahrt-Industrie, der Energietechnik (z.B. in Windkraftanlagen) und im Boots- und Schiffbau eingesetzt werden können. Die erfindungsgemäß verwendbaren reaktiven Zusammensetzungen sind umweltfreundlich, kostengünstig, weisen gute mechanische Eigenschaften auf, lassen sich einfach verarbeiten und zeichnen sich durch eine gute Wetterbeständigkeit wie auch durch ein ausgewogenes Verhältnis zwischen Härte und Flexibilität aus. Der Begriff Composite-Halbzeuge wird im Rahmen dieser Erfindung synonym zu den Begriffen Prepreg und Organoblech verwendet. Bei einem Prepreg handelt es sich in der Regel um eine Vorstufe für duroplastische Composite-Bauteile. Bei einem Organoblech handelt es sich normalerweise um eine entsprechende Vorstufe für thermoplastische Composite-Bauteile.

## Patentansprüche

1. Reversibel vernetzbare Formulierung, **dadurch gekennzeichnet, dass** die Formulierung mittels einer Hetero-Diels-Alder-Reaktion vernetzbar ist, dass die Formulierung eine Komponente A enthält, die mindestens zwei dienophile Doppelbindungen aufweist, wobei die Komponente A mindestens einmal die folgende Struktureinheit Z 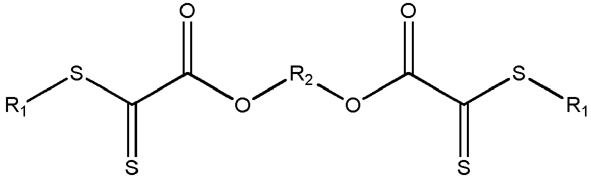 aufweist, wobei es sich bei R₁ um einen Alkyl- oder Alkylenrest mit 1 bis 20 Kohlenstoffatomen handelt, wobei der Alkylenrest mit weiteren der aufgezeigten Strukturen verbunden sein kann, und dass die Formulierung eine Komponente B enthält, die mindestens eine Dien-Funktionalität aufweist.

2. Formulierung gemäß Anspruch 1, **dadurch gekennzeichnet, dass** zumindest eine dieser beiden Komponenten A oder B mehr als zwei Funktionalitäten aufweist, dass mindestens eine der Komponenten A oder B als Polymer vorliegt, und dass die Formulierung bei Raumtemperatur vernetzbar ist und die Vernetzung bei einer höheren Temperatur zu mindestens 50% wieder rückgängig gemacht werden kann.

3. Formulierung gemäß Anspruch 1 oder 2, **dadurch gekennzeichnet, dass** es sich bei Komponente A um eine Verbindung mit mehreren der genannten Struktureinheiten Z handelt, und diese mit Alkylengruppen R₁, die zwischen 1 und 5 Kohlenstoffatome aufweisen, miteinander verbunden sind, und dass der R₂ zwischen 2 und 10 Kohlenstoffatome aufweist.

4. Formulierung gemäß Anspruch 3, **dadurch gekennzeichnet, dass** es sich bei der Komponente A um die Verbindung 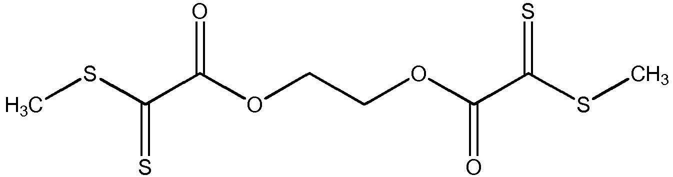 und/oder um die Verbindung 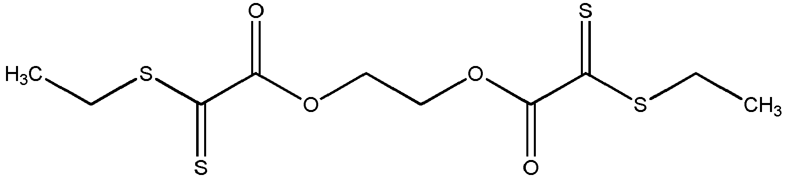 handelt.

5. Formulierung gemäß mindestens einem der Ansprüche 1 bis 4, **dadurch gekennzeichnet, dass** es sich bei der Komponente B um ein Polymer handelt.

6. Formulierung gemäß Anspruch 5, **dadurch gekennzeichnet, dass** es sich bei dem Polymer um Polyacrylate, Polymethacrylate, Polystyrole, Mischpolymerisate aus Acrylaten, Methacrylaten und/oder Styrolen, Polyacrylnitril, Polyether, Polyester, Polymilchsäuren, Polyamide, Polyesteramide, Polyurethane, Polycarbonate, amorphe oder teilkristalline Poly-α-Olefine, EPDM, EPM, hydrierte oder nicht hydrierte Polybutadiene, ABS, SBR, Polysiloxane und/oder Block-, Kamm- und/oder Sterncopolymeren dieser Polymere handelt.

7. Formulierung gemäß mindestens einem der Ansprüche 1 bis 5, **dadurch gekennzeichnet, dass** es sich bei der Komponente B um eine der folgenden Verbindungen 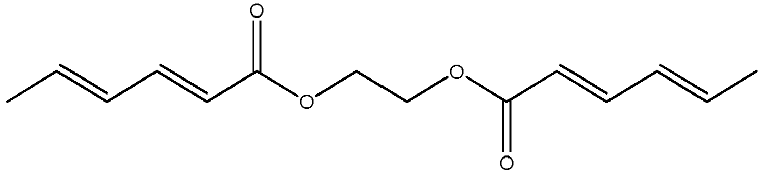 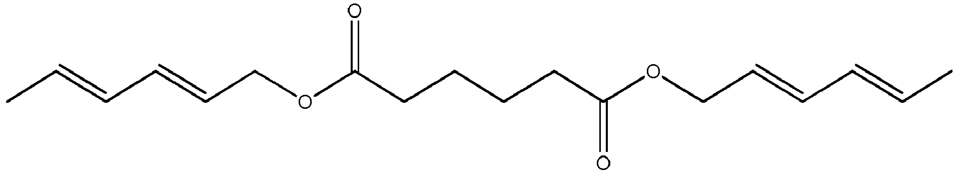 handelt.

8. Formulierung gemäß mindestens einem der Ansprüche 1 bis 6, **dadurch gekennzeichnet, dass** es sich bei der Komponente B um ein Polymer, welches unter Copolymerisation mindestens einer der folgenden Comonomere 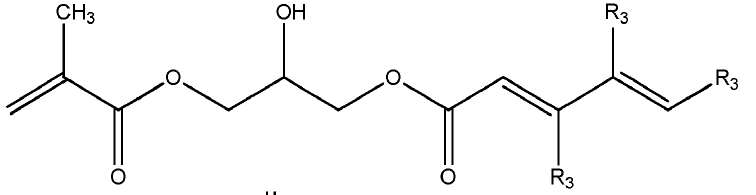 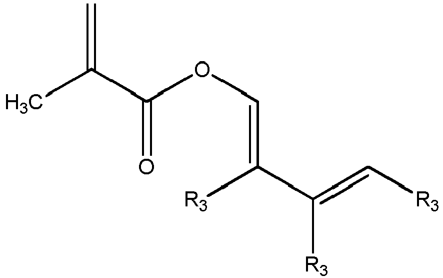 erhalten wurde, handelt, wobei es sich bei den Resten R3 um gleiche oder verschiedene Reste handeln kann, wobei es sich bei R3 um Wasserstoff und/oder um einen Alkylrest mit 1 bis 10 Kohlenstoffatomen handelt.

9. Formulierung gemäß mindestens einem der Ansprüche 1 bis 6, **dadurch gekennzeichnet, dass** es sich bei der Komponente B um ein Polyamid, einen Polyester oder ein Polycarbonat, aufweisend mindestens eine Dienfuntkionalität, handelt.

10. Formulierung gemäß Anspruch 1 oder 2, **dadurch gekennzeichnet, dass** Komponente A genau eine Struktureinheit Z und Komponente B genau eine Diengruppe aufweist.

11. Verfahren zur reversiblen Vernetzung, **dadurch gekennzeichnet, dass** eine Formulierung gemäß mindestens einem der Ansprüche 1 bis 10 mittels einer Hetero-Diels-Alder-Reaktion bei Raumtemperatur vernetzt wird und bei einer höheren Temperatur mindestens 50% der Vernetzungsstellen mittels einer Retro-Hetero-Diels-Alder-Reaktion wieder gelöst werden.

12. Verfahren gemäß Anspruch 11, **dadurch gekennzeichnet, dass** bei einer Temperatur oberhalb von 80 °C bevorzugt innerhalb von 5 min mindestens 90 % der Formulierung in einem für die Formulierung vor der Vernetzung geeigneten Lösungsmittel wieder löslich sind.

13. Verfahren gemäß Anspruch 11 bis 12, **dadurch gekennzeichnet, dass** die Vernetzung nach dem Vermischen der Komponenten A und B innerhalb von 2 min erfolgt.

14. Verfahren gemäß zumindest einem der Ansprüche 11 bis 13, **dadurch gekennzeichnet, dass** die Vernetzung nach dem Vermischen der Komponenten A und B mit einem Vernetzungskatalysator innerhalb von 2 min erfolgt.

15. Verwendung einer Formulierung gemäß zumindest einem der Ansprüche 1 bis 10 in Klebstoffen, Dichtstoffen, Formmassen, Schäumen, Lacken, Farben, Beschichtungen oder Tinten.

16. Verwendung einer Formulierung gemäß zumindest einem der Ansprüche 1 bis 10 in Composites für Anwendungen im Bereich der Bau-, der Automobil-, der Luft- und Raumfahrt-Industrie, der Energietechnik, wie Windkraftanlagen, im Boots- oder Schiffbau.
